# EUROPEAN PATENT APPLICATION

(11) **EP 1 371 369 A1**
(43) Date of publication of application: **17.12.2003**
(21) Application number: 03077579.5
(22) Date of filing: 02.03.2000
(51) Int. Cl.: A61K 31/445, A61K 9/06, A61K 47/02

(54) **Compositions comprising remifentanil**

(30) Priority: 04.03.1999 GB 9904931
(62) Divisional of application: 00912529.5
(71) Applicant: GLAXO GROUP LIMITED, Greenford, Middlesex UB6 ONN (GB)
(72) Inventor: Galante, Leonard John, Research Triangle Park, NC 27709 (US); Heiman, Shirley Ann, Research Triangle Park, NC 27709 (US); Igo, David H., Research Triangle Park, NC 27709 (US); Nguyen, Ngoc-Anh Thi, Research Triangle Park, NC 27709 (US)
(74) Representative: Hockley, Siân Catherine

(57) **Abstract**

The present invention provides a lower-dose of remifentanil in a stable formulation suitable for use by general practitioners. Specifically, the present invention provides an aqueous composition prepared for lyophilization comprising remifentanil (or a pharmaceutically acceptable salt or solvate thereof), sodium chloride and hydrochloric acid. The invention further provides a lyophilized solid form composition comprising remifentanil (or a pharmaceutically acceptable salt or solvate thereof) and sodium chloride. The invention yet also provides a lyophilized solid form composition reconstituted into a solution suitable for IV administration comprising remifentanil (or a pharmaceutically acceptable salt or solvate thereof) and sodium chloride.

## Description

The present invention relates to compositions containing N-phenyl-N-(4-piperidinyl)amides. In particular, the present invention relates to compositions containing 3-[4-methoxycarbonyl]-4-[(1-oxopropyl)phenylamino]-1-piperidine]propanoic acid, methyl ester (remifentanil).

The hydrochloric salt of remifentanil is commercially available from Glaxo Wellcome Inc. under the tradename ULTIVA® (remifentanil hydrochloric) for Injection. U.S. 5,866,591 covers various formulations of ULTIVA® for Injection containing glycine and U.S. 5,599,938 covers processes for synthesizing intermediate(s) of remifentanil, both incorporated by reference herein. Use of remifentanil to provide analgesia is described in U.S. 5,019,583, and Feldman PL, James MK, Brackeen MF, Bilotta JM, Schuster SV, Lahey AP, Lutz MW, Johnson MR, Leighton HJ "Design, Synthesis and Pharmacological Evaluation of Ultrashort to Long-Acting Opioid Analgesics," *J Med Chem* 34:2202-2208 (1991) incorporated by reference herein. Use of remifentanil to provide anesthesia and create conscious sedation is described in U.S. 5,466,700 incorporated by reference herein.

ULTIVA® is a µ-opioid agonist indicated for IV administration as (1) an analgesic agent for use during the induction and maintenance of general anesthesia for inpatient and outpatient procedures, (2) for continuation as an analgesic into the immediate postoperative period under the direct supervision of an anesthesia practitioner in a postoperative anesthesia care unit or intensive care setting, and (3) as an analgesic component of monitored anesthesia care. ULTIVA® is chemically designated as a 3-[4-methoxycarbonyl-4-[(1-oxopropyl)phenylamino]-1-piperidine]propanoic acid methyl ester, hydrochloric salt, C₂₀H₂₈N₂0₅•HCl, with a molecular weight of 412.91 and has the following chemical structure:

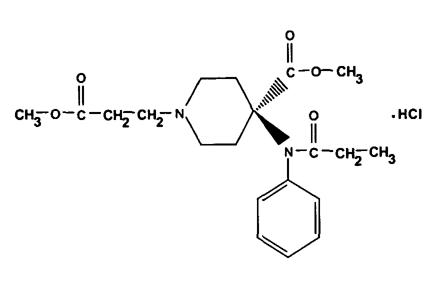

ULTIVA® is formulated as a sterile, nonpyrogenic, preservative-free, white to off-white lyophilized powder for intravenous (IV) administration after reconstitution and dilution. It is packaged in vials which contain 1, 2, or 5 mg of remifentanil base in the form of its hydrochloride salt; 15 mg glycine; and hydrochloric acid to buffer the solutions to a nominal pH of 3 after reconstitution. When reconstituted as directed, solutions of ULTIVA® are clear and colorless and contain remifentanil hydrochloric (HCI) equivalent to 1 mg/mL of remifentanil base. The pH of reconstituted solutions of ULTIVA® ranges from 2.5 to 3.5.

Remifentanil contains two alkyl esters, one sterically hindered and the other sterically unhindered. The sterically unhindered ester is believed to be susceptible to aqueous hydrolysis and hydrolysis by esterases in blood and tissues. The major hydrolysis product is the monocarboxylic acid which is significantly less potent than remifentanil. Thus the rapid inactivation of remifentanil is caused by hydrolysis of the sterically unhindered ester. Rapid inactivation provides the following advantages for using remifentanil in anesthetic/analgesic applications: (1) ultrashort duration of action (2) less variability in recovery rate even with differences in hepatic function (3) no accumulation of drug during repeated bolus doses or infusion; and (4) more rapid recovery.

Despite this advantageous profile, general practitioners (versus anesthesiologists) are in need of an anesthetic more suitable for short painful procedures on an outpatient basis. Because of the rapid onset of action of remifentanil, accurate dosing and monitored care are important in order to avoid adverse effects. This is not always suitable for outpatient procedures performed by general practitioners. In order to administer remifentanil in short painful procedures on an outpatient basis, a lower dose formulation than that currently marketed is needed. However, simple reduction in strength of the currently available formulations does not yield desired stability of the product. Moreover, while remifentanil's susceptibility to rapid hydrolysis results in many of its benefits, it also makes it very difficult to arrive at a stabilized formulation. A lower-dose formulation would be more susceptible to hydrolysis than the currently marketed formulation.

The present invention provides a lower-dose of remifentanil in a stable formulation suitable for use by general practitioners. Specifically, the present invention provides an aqueous composition prepared for lyophilization comprising remifentanil (or a pharmaceutically acceptable salt or solvate thereof), sodium chloride and hydrochloric acid. The invention further provides a lyophilized solid form composition comprising remifentanil (or a pharmaceutically acceptable salt or solvate thereof) and sodium chloride. The invention yet also provides a lyophilized solid form composition reconstituted into a solution suitable for IV administration comprising remifentanil (or a pharmaceutically acceptable salt or solvate thereof) and sodium chloride.

A first aspect of the invention is an aqueous composition prepared for lyophilization comprising remifentanil (or a pharmaceutically acceptable salt or solvate thereof), sodium chloride and hydrochloric acid.

A particular embodiment of the first aspect of the invention is an aqueous composition prepared for lyophilization comprising remifentanil (or a pharmaceutically acceptable salt or solvate thereof), sodium chloride and hydrochloric acid wherein said composition has a pH of about 1 to 5.

Another particular embodiment of the first aspect of the invention is an aqueous composition prepared for lyophilization comprising remifentanil (or a pharmaceutically acceptable salt or solvate thereof), sodium chloride and hydrochloric acid wherein said composition has a pH of about 2 to 4.

Another particular embodiment of the first aspect of the invention is an aqueous composition prepared for lyophilization comprising remifentanil (or a pharmaceutically acceptable salt or solvate thereof), sodium chloride and hydrochloric acid wherein said composition has a pH of about 2.75 to 3.25.

Another particular embodiment of the first aspect of the invention is an aqueous composition prepared for lyophilization comprising about 0.01 mg/ml to 0.5 mg/ml of remifentanil (or a pharmaceutically acceptable salt or solvate thereof), about 9 mg/ml to 81 mg/ml sodium chloride and about 0.0001 to 0.01 N hydrochloric acid (pH 2-4).

Another particular embodiment of the first aspect of the invention is an aqueous composition prepared for lyophilization comprising about 0.025 mg/ml to 0.2 mg/ml of remifentanil (or a pharmaceutically acceptable salt or solvate thereof) and about 36 mg/ml to 72 mg/ml sodium chloride and about 0.001 to 0.01 N hydrochloric acid (pH 2-3).

Another particular embodiment of the first aspect of the invention is an aqueous composition prepared for lyophilization comprising 0.1 mg/ml of remifentanil (or a pharmaceutically acceptable salt or solvate thereof) and 72 mg/ml sodium chloride and 0.001 N hydrochloric acid (pH 3).

A second aspect of the invention is a lyophilized solid form composition comprising remifentanil (or a pharmaceutically acceptable salt or solvate thereof) and sodium chloride. In the following embodiments of the second aspect of the invention, the concentration of hydrochloric acid is reduced to trace amounts. Trace amounts being equal to about 1% or less of the concentration of hydrochloric acid in the corresponding embodiments of the first aspect of the invention (aqueous composition prepared for lyophilization comprising remifentanil (or a pharmaceutically acceptable salt or solvate thereof), sodium chloride and hydrochloric acid)).

A particular embodiment of the second aspect of the invention is a lyophilized solid form composition comprising about 0.01 mg to 0.5 mg remifentanil (or a pharmaceutically acceptable salt or solvate thereof) and sodium chloride.

Another particular embodiment of the second aspect of the invention is a lyophilized solid form composition comprising about 0.025 mg to 0.2 mg of remifentanil (or a pharmaceutically acceptable salt or solvate thereof) and sodium chloride.

Another particular embodiment of the second aspect of the invention is a lyophilized solid form composition comprising about 0.1 mg of remifentanil (or a pharmaceutically acceptable salt or solvate thereof) and sodium chloride.

Another particular embodiment of the second aspect of the invention is a lyophilized solid form composition comprising about 0.01 mg to 0.5 mg of remifentanil (or a pharmaceutically acceptable salt or solvate thereof) and about 9 mg to 81 mg sodium chloride.

Another particular embodiment of the second aspect of the invention is a lyophilized solid form composition comprising about 0.025 mg to 0.2 mg of remifentanil (or a pharmaceutically acceptable salt or solvate thereof) and about 36 mg to 72 mg sodium chloride.

Another particular embodiment of the second aspect of the invention is a lyophilized solid form composition comprising about 0.1 mg of remifentanil (or a pharmaceutically acceptable salt or solvate thereof) and about 72 mg sodium chloride.

Another particular embodiment of the second aspect of the invention is a lyophilized solid form composition comprising about 0.1 mg of remifentanil (or a pharmaceutically acceptable salt or solvate thereof) and about 72 mg sodium chloride having shelf-life stability equal to or less than about 10% degradation over 18 months at or below 25°C.

Another particular embodiment of the second aspect of the invention is a lyophilized solid form composition comprising about 0.1 mg of remifentanil (or a pharmaceutically acceptable salt or solvate thereof) and about 72 mg sodium chloride having shelf-life stability equal to or less than about 10% degradation over 2 years at or below 25°C.

A third aspect of the invention is a lyophilized solid form composition reconstituted into a solution suitable for IV administration comprising remifentanil (or a pharmaceutically acceptable salt or solvate thereof) and sodium chloride.

Lyophilized compositions of the invention may be reconstituted with the following fluids (diluents) in order to achieve desired concentrations of remifentanil (or a pharmaceutically acceptable salt or solvate thereof) prior to IV administration: Sterile Water for Injection (USP), 5% Dextrose Injection (USP), 5% Dextrose and 0.9% sodium Chloride Injection (USP) 0.9% sodium Chloride Injection (USP) or 0.45% sodium Chloride Injection (USP). The volume of the diluent may be varied.

Lyophilized solid form compositions reconstituted into solutions suitable for IV administration may be administered using a rapid intravenous bolus on the order of about 2 seconds.

A particular embodiment of the third aspect of the invention is a lyophilized solid form composition reconstituted into a solution suitable for IV administration comprising an amount of remifentanil (or a pharmaceutically acceptable salt or solvate thereof) of about 0.01 mg to 0.5 mg and an amount of sodium chloride of about 9 mg to 81 mg.

Another particular embodiment of the third aspect of the invention is a lyophilized solid form composition reconstituted into a solution suitable for IV administration comprising an amount of remifentanil (or a pharmaceutically acceptable salt or solvate thereof) of about 0.025 mg to 0.2 mg and an amount of sodium chloride of about 36 mg to 72 mg.

Another particular embodiment of the third aspect of the invention is a lyophilized solid form composition reconstituted into a solution suitable for IV administration comprising an amount of remifentanil (or a pharmaceutically acceptable salt or solvate thereof) of about 0.1 mg and an amount of sodium chloride of about 72 mg.

Another particular embodiment of the third aspect of the invention is a lyophilized solid form composition reconstituted into about 1 ml to 9 ml of Sterile Water for Injection comprising an amount of remifentanil (or a pharmaceutically acceptable salt or solvate thereof) of about 0.01 mg to 0.5 mg and an amount of sodium chloride of about 9 mg to 81 mg.

Another particular embodiment of the third aspect of the invention is a lyophilized solid form composition reconstituted into about 4 ml to 8 ml of Sterile Water for Injection comprising an amount of remifentanil (or a pharmaceutically acceptable salt or solvate thereof) of about 0.025 mg to 0.2 mg and an amount of sodium chloride of about 36 mg to 72 mg.

Another particular embodiment of the third aspect of the invention is a lyophilized solid form composition reconstituted into about 8 ml of Sterile Water for Injection comprising an amount of remifentanil (or a pharmaceutically acceptable salt or solvate thereof) of about 0.1 mg and an amount of sodium chloride of about 72 mg.

Another particular embodiment of the third aspect of the invention is a lyophilized solid form composition reconstituted into a solution suitable for IV administration as described in any of the above embodiments of the third aspect wherein the pH of said solution is about 4 to 6.

Another particular embodiment of the third aspect of the invention is a lyophilized solid form composition reconstituted into a solution suitable for IV administration as described in any of the above embodiments of the third aspect wherein the pH of said solution is about 5.

A fourth aspect of the invention comprises formulations described in the first three aspects with the following embodiments independently or cooperatively employed.

A first particular embodiment of the fourth aspect of the invention comprises formulations described in the first three aspects wherein said formulations are substantially free of glycine.

Another particular embodiment of the fourth aspect of the invention comprises formulations described in the first three aspects wherein said composition is substantially free of phosphoric acid.

The term substantially free as used above means less than 5% and preferably less than 1%.

Another particular embodiment of the fourth aspect of the invention comprises formulations described in the first three aspects wherein remifentanil is provided as its hydrochloric salt.

A fifth aspect of the invention comprises a method of providing analgesia using a lyophilized solid form composition comprising remifentanil (or a pharmaceutically acceptable salt or solvate thereof) and sodium chloride reconstituted into a solution suitable for IV administration in an amount effective for analgesia but not sufficient to induce anesthesia. It is understood by those skilled in the art that other anesthesia-inducing agents may be co-administered with the compound of this invention in the course of their clinical application. Thus "an amount effective for analgesia but not sufficient to induce anesthesia" refers to an amount of remifentanil (or a pharmaceutically acceptable salt or solvate thereof) effective for analgesia but not sufficient to induce anesthesia if administered in the absence of other agents capable of inducing anesthesia.

Amounts of remifentanil (or a pharmaceutically acceptable salt or solvate thereof) effective for analgesia but not sufficient to induce anesthesia range from about 0.01 mg to 0.5 mg, particularly about 0.025 mg to 0.2 mg and more particularly about 0.1 mg.

Lyophilized solid form compositions reconstituted into a solution suitable for IV administration may be administered using a rapid intravenous bolus on the order of about 2 seconds.

Amounts or concentrations of remifentanil (or pharmaceutically acceptable salts or solvates thereof) referred to herein are calculated based upon the molecular weight of remifentanil free base.

### Examples

Prepare 0.001 N hydrochloric acid (pH 3) by appropriate dilution from Diluted Hydrochloric Acid USP. Partially fill the manufacturing vessel with the appropriate volume of 0.001 N hydrochloric acid (about 2/3 of final volume). Weigh 72 gm sodium chloride and dissolve. Weigh an amount of remifentanil hydrochloric equivalent to 100 mg of remifentanil free base and dissolve. Dilute to volume or weight of 1,000 ml with 0.001 N hydrochloric acid. Filter bulk solution with Millipore .22 micron filter. Fill each vial with 1 mL of solution and partially stopper. The formulation in each vial contains 0.1 mg remifentanil, 72 mg sodium chloride with appropriate volume of 0.001 N hydrochloric acid (pH 3) to final volume of 1 mL.

Following is a general description of the lyophilization methods employed. Several lyophilizers may be employed to make compositions of the present invention (FTS System (chamber/micro processor #0052449, condenser # 0052450) Freeze Dryer or Biolafitte (#92636) Freeze Dryer). Cool the shelves to 5°C. Load the shelves with 1 ml vials referred to above and then cool to approximately -45°C. Hold the shelves at -45°C for approximately 1.5 hours. Evacuate the chamber to 125 microns. Warm the shelves to -10°C in about one hour. Maintain shelves at - 10°C for approximately 6 hours. Warm the shelves to 25°C in about half an hour. Hold the shelves' temperature at 25°C for approximately 4 hours. Bleed the chamber with nitrogen to atmospheric pressure. Stopper the vials. The formulation is presented in a lyophilized form for shelf life stability. This lyophilized formulation was found to be stable (e.g., less than 10% degradation) for 1 month at 60°C. Stability at 60°C for 1 month is predictive of stability at 25°C for 24 months.

**Table 1.**

| **Typical Lyophilization Cycles FTS and Biolafitte Freeze-Dryers** | | | | | | | | |
|---|---|---|---|---|---|---|---|---|
| | **FTS** | | | | **Biolafitte** | | | |
| Step | Pressure (micron) | Initial Temp (°C) | Final Temp (°C) | Time (hours) | Pressure (micron) | Initial Temp (°C) | Final Temp (°C) | Time (hours) |
| Loading | atm | +25 | +25 | 0.5 | atm | +5 | +5 | 0.5 |
| Freezing | atm | +25 | -45 | 1.0 | atm | +5 | -45 | 0.5 |
| Freezing | atm | -45 | -45 | 1.5 | atm | -45 | -45 | 1.5 |
| Primary Drying | 300 | -45 | 0 | 1.0 | 125 | -45 | -10 | 1.0 |
| Primary Drying | 300 | 0 | 0 | 6.0 | 125 | -10 | -10 | 6.0 |
| Secondary Drying | 300 | 0 | +25 | 0.5 | 125 | -10 | +25 | 0.5 |
| Secondary Drying | 300 | +25 | +25 | 4.0 | 125 | +25 | +25 | 4.0 |
| Stoppering | atm | +25 | +25 | N/A | atm | +25 | +25 | N/A |
| Total Run Time | | | | 14.5 | | | | 14.0 |

Reconstitute with 8 mL of Water for Injection to provide an isotonic solution (0.9% sodium chloride) with 0.0125 mg/mL remifentanil suitable for IV administration.

## Claims

1. An aqueous composition prepared for lyophilization comprising remifentanil (or a pharmaceutically acceptable salt or solvate thereof), sodium chloride and hydrochloric acid wherein said composition is substantially free of glycine.

2. An aqueous composition prepared for lyophilization comprising about 0.01 mg/ml to 0.5 mg/ml of remifentanil (or a pharmaceutically acceptable salt or solvate thereof), about 9 mg/ml to 81 mg/ml sodium chloride and about 0.0001 to 0.01 N hydrochloric acid.

3. An aqueous composition prepared for lyophilization comprising about 0.025 mg/ml to 0.2 mg/ml of remifentanil (or a pharmaceutically acceptable salt or solvate thereof) and about 36 mg/ml to 72 mg/ml sodium chloride and about 0.001 to 0.01 N hydrochloric acid.

4. An aqueous composition prepared for lyophilization comprising 0.1 mg/ml of remifentanil (or a pharmaceutically acceptable salt or solvate thereof) and 72 mg/ml sodium chloride and 0.001 N hydrochloric acid.

5. The composition of claim 2 that is substantially free of glycine.

6. The composition of claim 3 that is substantially free of glycine.

7. The composition of claim 4 that is substantially free of glycine.

8. The composition of claim 3 wherein said pharmaceutically acceptable salt of remifentanil is remifentanil hydrochloride and wherein said composition is substantially free of glycine and phosphoric acid.

9. A lyophilized solid form composition comprising about 0.01 mg to 0.5 mg of remifentanil (or a pharmaceutically acceptable salt or solvate thereof) and about 9 mg to 81 mg sodium chloride.

10. A lyophilized solid form composition comprising about 0.025 mg to 0.2 mg of remifentanil (or a pharmaceutically acceptable salt or solvate thereof) and about 36 mg to 72 mg sodium chloride.

11. A lyophilized solid form composition comprising about 0.1 mg of remifentanil (or a pharmaceutically acceptable salt or solvate thereof) and about 72 mg sodium chloride.

12. A lyophilized solid form composition comprising about 0.1 mg of remifentanil (or a pharmaceutically acceptable salt or solvate thereof) and about 72 mg sodium chloride having shelf-life stability equal to or less than about 10% degradation over 18 months at or below 25 C.

13. The composition of claim 9 that is substantially free of glycine.

14. The composition of claim 11 that is substantially free of glycine.

15. The composition of claim 12 that is substantially free of glycine.

16. A lyophilized solid form composition reconstituted into a solution suitable for IV administration comprising an amount of remifentanil (or a pharmaceutically acceptable salt or solvate thereof) of about 0.01 mg to 0.5 mg and an amount of sodium chloride of about 9 mg to 81 mg.

17. A lyophilized solid form composition reconstituted into a solution suitable for IV administration comprising an amount of remifentanil (or a pharmaceutically acceptable salt or solvate thereof) of about 0.025 mg to 0.2 mg and an amount of sodium chloride of about 36 mg to 72 mg.

18. A lyophilized solid form composition reconstituted into a solution suitable for IV administration comprising an amount of remifentanil (or a pharmaceutically acceptable salt or solvate thereof) of about 0.1 mg and an amount of sodium chloride of about 72 mg.

19. A method of providing analgesia using a lyophilized solid form composition reconstituted into a solution suitable for IV administration in an amount effective for anlagesia but not sufficient to induce anesthesia using a composition of claim 16.

20. A method of providing analgesia using a lyophilized solid form composition reconstituted into a solution suitable for IV administration in an amount effective for analgesia but not sufficient to induce anesthesia using a composition of claim 18.

21. A lyophilized solid form composition comprising remifentanil (or a pharmaceutically acceptable salt or solvate thereof) and sodium chloride, wherein said composition is substantially free of glycine.

22. A lyophilized solid form composition as claimed in claim 21 wherein the ratio of remifentanil (or a pharmaceutically acceptable salt or solvate thereof) and sodium chloride is within the range of 1:18 to 1:8100 parts by weight.

23. A composition as claimed in claim 22 wherein the ratio of remifentanil (or a pharmaceutically acceptable salt or solvate thereof) and sodium chloride is within the range of 1:18 to 1:3000 parts by weight.

24. A composition as claimed in claim 23 wherein the ratio of remifentanil (or a pharmaceutically acceptable salt or solvate thereof) and sodium chloride is about 1:720 parts by weight.

25. A method of providing analgesic using a lyophilized solid form composition as claimed in any of claims 21-24, reconstituted into a solution suitable for IV administration in an effective amount for analgesia but not sufficient to induce anesthesia.
